# EUROPEAN PATENT SPECIFICATION

(11) **EP 3 422 856 B1**
(45) Date of publication and mention of the grant of the patent: **14.06.2023**
(21) Application number: 17760372.7
(22) Date of filing: 02.03.2017
(51) Int. Cl.: A61K 33/14, A61K 35/00, A01N 59/08, A01K 61/13, A61K 35/08, A61K 9/00, A61P 33/00

(54) **PREPARATION CONTAINING SEA WATER AND AN ADDED POTASSIUM COMPOUND**
PRÄPARAT MIT MEERWASSER UND ZUGESETZTER KALIUMVERBINDUNG
PRÉPARATION CONTENANT DE L'EAU ET UN COMPOSÉ SUPPLÉMENTAIRE DE POTASSIUM

(30) Priority: 04.03.2016 NO 20160382
(43) Date of publication of application: 09.01.2019
(73) Proprietor: Vestland Pharma AS, 6823 Sandane (NO)
(72) Inventor: KANDAL, Inge Arne, 6770 Nordfjordeid (NO)
(74) Representative: Håmsø Patentbyrå AS
(86) International application number: PCT/NO2017/050059
(87) International publication number: WO 2017/150988

(56) References cited:
- EP-A1- 2 174 648
- EP-A1- 2 912 956
- WO-A1-98/53807
- WO-A1-2014/177594
- WO-A1-2016/021523
- CN-A- 103 479 526
- FR-A5- 2 082 097
- JP-A- S5 810 509
- JP-A- H05 117 158
- JP-B2- 4 976 006
- NO-A1- 20 131 294
- NO-A1- 20 131 294
- US-A1- 2003 232 366
- US-B1- 6 855 325
- BRICKNELL I. R. ET AL.: "Effect of environmental salinity of sea lice Lepeophtherius salmonis settlement success", DISEASES OF AQUATIC ORGANISMS, vol. 71, 2006, pages 201-212, XP055401585,
- JONES S. ET AL.: BIOLOGY OF SEA LICE, LEPEOPHTERIRUS SALMONIS AND CALIGUS SPP., March 2015 (2015-03), page 4, XP055414722, WESTERN AND EASTERN CANADA
- SACKVILL S. ET AL.: "Pink Salmon (Oncorhynchus gorbuscha) osmoregulatory development plays a key role in sea louce (Lepeophtheirus salmonis) tolerance", CANADIAN J. FISH. AQUAT. SCI., vol. 68, no. 6, 1 June 2011 (2011-06-01), pages 1087-1096, XP055559273, DOI: 10.1139/f2011-037
- MARSHALL W. S ET AL.: "6 Ion transport, osmoregulation , and acid-base balace", THE PHYSIOLOGY OF FISHES, 2005, pages 177-230, XP055414734,
- EWANS D. ET AL.: "The effect of external potassium ions on the electrical potential measured across the gills of the teleost, Dormitator Maculatus", J. EXP. BIOL., vol. 61, 1974, pages 277-283, XP055414737,

## Description

The invention relates to a preparation containing sea water to which a potassium compound is added. The preparation consists of a compound for use as a medicament for the topical treatment of fish having an external parasite. More specifically, the parasite may be a crustacean parasite, and especially a marine crustacean parasite. The invention relates, more specifically, to a preparation for topical treatment in the form of a bath treatment for fish. The preparation comprises sea water to which there is added the potassium compound such that the amount of potassium ions in the preparation is increased in relation to the amount of potassium ions naturally present in the sea water.

The farming of fish, especially in sea water, is plagued by parasitic diseases. In particular, infections by so-called salmon lice on salmonoids are well known. Such infections may be lethal to the salmonoids if they are not treated.

Salmon louse will be used herein referring to different types of external, marine crustacean parasites belonging to the family *Caligidae, inter alia* Scottish lice (*Caligus elongatus*) and real salmon lice (*Lepeophtheirus salmonis*)*.* Other species are known as well. Like other crustaceans, these ectoparasites grow by moulting, and they go through a total of 8 stages in their life cycle. From the egg, a free-swimming Nauplius I larva is first released. In *L. salmonis,* this is 0.5 mm long and the sizes below refer to salmon lice. The nauplius I grows into a nauplius II larva, 0.6 mm long, which in turn grows into a copepodid 0.7 mm long. It is the copepodid that constitutes the infectious stage as the copepodid will attach to a host. The first three stages may last several weeks, and the duration depends on the water temperature. In low water temperatures the larvae grow slowly.

After the copepodid has attached to the skin of the host, it moults again and develops through two chalimus stages, chalimus I - chalimus II. This stage is characterized by the parasite attaching to the skin of the host through a frontal filament. The parasite therefore cannot move around freely on the host. The parasite lives off fish mucus on the skin of the host. The parasite grows from 1.1 mm to 2.3 mm during the chalimus stage. After the chalimus II comes the pre-adult I stage in which the parasite is no longer stuck to the host by the frontal filament but can move freely on the skin of the host. Pre-adult I males are 3.4 mm long; the females are 3.6 mm long. The parasite grows on into the pre-adult II stage; the male is then 4.3 mm long, the female 5.2 mm long. The parasite then moults for the last time and reaches the adult stage. An adult male is 5-6 mm long; the female is 8-12 mm long. After mating, the adult female lets the eggs into two egg sacs. These may each contain several hundred eggs. The egg strings are released, the eggs hatch and the life cycle is then repeated.

It is as a freely moving pre-adult and adult that the parasite causes the greatest damage. Primarily, the parasite lives off mucus, but will, particularly when there are many lice on a fish, attack the skin layer so that the underlying muscular tissues and bone tissues become exposed. The fish is then vulnerable to attacks by bacteria and fungi, and the fish will have problems with its osmosis regulation. An extensive attack by salmon lice is lethal.

Salmonoids may be salmon (*Salmo salar*), rainbow trout (*Oncorhynchus mykiss*) and Pacific salmon (*Oncorhynchus* spp.).

In fish-farming, at least three different forms of treatment of salmon lice are known. The first form of treatment that was developed consisted of bathing the fish in a solution of a treatment agent and sea water. Bathing agents include hydrogen peroxide, pyrethroids and organophosphates. One drawback of bath treatment is that a skirt in the form of a tarpaulin must be put around the net cage in which the fish are in, in order to limit the volume. In addition, the seine bottom is lifted up to further limit the volume. The treatment agent is then added to the sea water and the fish swim around in the treatment solution for a prescribed time. It is heavy and laborious to treat the fish in this way, and the treatment may have to be repeated several times. The fish also become stressed by the crowding when the seine bottom is lifted.

The second known form of treatment is the admixture of a parasite agent into the feed that the fish eat. Such known veterinary-medicinal pharmaceuticals include an admixture of emamectin benzoate and chitin synthesis inhibitors. For the farmer, this is a not a very laborious method because the pharmaceutical is provided in the feed that the fish are going to consume. The drawback is that a medicated feed is a medicinal preparation which is produced on dedicated production lines at the feed producers' premises. Such a medicinal feed is expensive.

The third known form of treatment is the use of so-called cleaner fish. In this relation it has turned out that lumpfish (*Cyclopterus lumpus*) and species of the wrasse family *Labridae* are well suited.

One drawback of therapeutic agents either in baths or in feeds is that, over time, the parasite develops resistance to these agents, and the agents therefore become less effective. When therapeutic pharmaceuticals are used, a withdrawal period from the treatment to the slaughtering of the fish is to be set to ensure that the fish meat will not contain the chemical or that the amount of chemical is below a set level. The third method which includes the use of cleaner fish is an alternative to pharmaceuticals, and the development of resistance is not a problem. Wrasses will remove parasites which are easy to see and will, in the main, remove pre-adult and adult parasites. The drawback of the use of wrasses is that it is difficult to make them spend the winter in the net cages together with the salmonoids; they may escape through the seine wall as they are substantially smaller than the salmonoids, and they can damage the salmonoids by so-called eye-biting.

It is known that salmon lice cannot survive long in water with low salinity. Patent document NO338812B1 discloses that chemicals and pharmaceuticals may increase the therapeutic effect of freshwater on salmon lice. Such chemicals and pharmaceuticals include KCI, isoeugenol and pyrethroids. The inventors have found that a drawback of using KCl dissolved in freshwater may be that the therapeutic margin between the host and the parasite is small.

There is thus a need for alternatives to today's methods of controlling crustacean parasites in fish-farming, or at least some supplementary methods.

By sea water is meant, in what follows, water having a salinity of between 2.0 % and 4.0 %, also stated as a salinity of between 20 ppt and 40 ppt, and having a composition identical to that of natural sea water or a composition resembling that of natural sea water. Natural sea water with a salinity of 3.5 % has a composition of ions in which the most important ions comprise 1.94 % (wt/wt) chlorine; 1.08 % (wt/wt) sodium; 0.13 (wt/wt) magnesium; 0.09 % (wt/wt) sulphur; 0.04 (wt/wt) calcium; 0.04 % (wt/wt) potassium; and 0.01 % (wt/wt) bromine. Minor deviations may occur. Common sea water has a salinity of between 30 ppt and 38 ppt. The amount of ions decreases or increases proportionally to the total salinity of the sea water.

It is within the invention to thin the sea water with fresh water so that the salinity will be between 20 ppt and 34 ppt. The amount of ions decreases or increases proportionally to the total salinity of the sea water. The sea water may be thinned with freshwater in a tank in a fish carrier.

It is also within the invention to add extra salt to the sea water such that the salinity will be between 35 ppt and 40 ppt. The extra salt may be sodium chloride or some other salt which does not contain potassium. The extra salt may be added to the sea water in a tank in a fish carrier.

Surprisingly, the inventors have found that potassium ions dissolved in sea water in specific doses have a good effect against salmon lice while, at the same time, there is a satisfactory therapeutic margin for the fish.

Potassium ions are naturally present in sea water. Sea water with a salinity of 3.5 % contains approximately 380 mg/l K⁺.

The invention has for its object to remedy or reduce at least one of the drawbacks of the prior art or at least provide a useful alternative to the prior art.

The object is achieved through features which are specified in the description below and in the claims that follow.

The invention relates, more specifically, to a preparation for use as a medicament for the topical treatment of fish infected with an external parasite, the preparation consists of sea water to which a potassium compound has been added, wherein the potassium compound is added in an amount corresponding to between 0.78 g of potassium ions per litre of said sea water and 1.21 g of potassium ions per litre of said sea water, and wherein the salinity of said sea water is between 20 ppt and 40 ppt. Said sea water is one of: natural sea water; natural sea water thinned with fresh water; and synthetic sea water containing at least chlorine, sodium magnesium, sulphur and potassium in approximately the same proportions as in natural sea water. By added it is meant that the potassium ions of the potassium compound come in addition to the potassium ions present in sea water.

The potassium compound may be a potassium salt. The potassium salt may be potassium chloride. The potassium salt may alternatively be potassium bromide, potassium iodide or potassium nitrate. The potassium compound may be other suitable potassium salts or other suitable potassium compounds liberating potassium ions on dissociation in sea water.

The potassium compound may be added to the sea water in an amount corresponding to between 0.78 g of potassium ions per litre of sea water and 1.21 g of potassium ions per litre of sea water. This is the equivalent of adding to the sea water approximately between 1.5 g of potassium chloride and 2.3 g of potassium chloride per litre of sea water.

The sea water may be natural sea water with a salinity of between 30 ppt and 38 ppt. The sea water may be a synthetic sea water containing at least chlorine, sodium, magnesium, sulphur and potassium in approximately the same proportions as in natural sea water. The sea water may comprise a sea water for which the measured salinity is stated rounded to 31 ppt, 32 ppt, 33 ppt, 34 ppt, 35 ppt, 36 ppt and 37 ppt. The sea water my further comprise a diluted sea water for which the measured salinity is stated rounded to 21 ppt, 22 ppt, 23 ppt, 24 ppt, 25 ppt, 26 ppt, 27 ppt, 28 ppt and 29 ppt. The sea water may further comprise a sea water and an added salt, for which the measured salinity is stated rounded to 39 ppt.

The external parasite may consist of a crustacean parasite. The crustacean parasite may consist of a *Lepeophtheirus* spp. or a *Caligus* spp.). The crustacean parasite may consist of a salmon louse (*Lepeophtheirus salmonis*). The fish may consist of a marine fish. The fish may consist of a salmonoid, such as salmon (*Salmo salar*), rainbow trout (*Oncorhynchus mykiss*) or a Pacific salmon (*Oncorhynchus* spp.).

The preparation may be produced by mixing sea water and the potassium compound in a tank in a fish carrier.

The preparation for use as a medicament may be applied topically for a period of at least 45 minutes. The topical application may take place through a bath treatment of the fish in the preparation. The preparation may be applied topically for a period of at least 60 minutes. The preparation may be applied topically for a period of between 45 minutes and 300 minutes. The preparation may be applied topically for a period of between 60 minutes and 300 minutes. The preparation may be applied topically for a period limited to 360 minutes. The preparation may be applied topically for a period limited to 300 minutes.

In what follows, examples of preferred embodiments are described.

For the trials, sea water with a salinity of 34.5 ppt was used. The sea-water temperature varied between 9.1 and 9.8 °C. The oxygen content varied between 93 and 98 % of full saturation.

Full-strength sea water with a salinity of 35 ppt contains approximately 380 mg/l K⁺ and approximately 19,400 mg/l Cl⁻.

### Example 1

The preparation for use as a medicament for the topical treatment of fish was sea water with an addition of 1.7 g KCl per litre of sea water. This is the equivalent of an addition of 0.89 g of potassium and 0.81 g of chlorine per litre of sea water. The salinity in the trial was thereby 36.2 ppt. The preparation solution of the trial contained approximately 1,266 mg/l K⁺ and 19,535 mg/l Cl⁻.

The trial was carried out with 10 salmon (*Salmo salar*)*.* The salmon weighed between 254 and 340 g. The average weight was 294 g.

The salmon were infected with adult female lice and male lice, altogether 195 lice. There was thus an average of 19 lice per salmon.

A vessel was filled with 80 l of sea water. The water was supplied with pressurized air from one nozzle. No currents were set up in the vessel. 136 g KCl was dissolved in the sea water so that there was an addition of 1.7 g KCl per litre.

The trial lasted 5 hours.

### Results

Salmon lice that had fallen off the fish were collected every 15 minutes.

The results are shown in Table 1.

**Table 1**

| Observation time (min) | Cumulative number fallen off (n) | Cumulative number fallen off (%) |
|---|---|---|
| 60 | 21 | 11 |
| 75 | 42 | 22 |
| 90 | 51 | 26 |
| 105 | 68 | 35 |
| 120 | 78 | 40 |
| 135 | 84 | 43 |
| 150 | 91 | 47 |
| 165 | 96 | 49 |
| 180 | 101 | 52 |
| 195 | 105 | 54 |
| 210 | 110 | 57 |
| 225 | 115 | 59 |
| 240 | 120 | 62 |
| 255 | 125 | 64 |
| 270 | 128 | 66 |
| 285 | 129 | 66 |
| 300 | 129 | 66 |

After the observation period of 300 minutes, remaining lice on the fish were counted. The number was 58. In addition, a further 7 salmon lice were found in the vessel.

The example shows that a preparation solution with 1.7 g/l of added potassium chloride in sea water has a considerable effect on the ability of salmon lice to stay on the fish.

### Example 2

The same quality of sea water and the same method as in Example 1 were used. A total of 14 salmon were infected with 117 adult male lice and female lice. The salmon weighed between 115 and 440 g. The average weight was 239 g. The preparation for use as a medicament for the topical treatment of fish had an addition of 1.8 g KCl per litre of sea water. This corresponds to an addition of 0.94 g of potassium and 0.86 g of chlorine per litre of sea water. The salinity in the trial was thereby 36.3 ppt. The preparation solution of the trial contained approximately 1,319 mg/l K⁺ and 19,585 mg/l Cl⁻.

The trial lasted 5 hours.

The results are shown in Table 2.

**Table 2**

| Observation time (min) | Cumulative number fallen off (n) | Cumulative number fallen off (%) |
|---|---|---|
| 60 | 0 | 0 |
| 75 | 1 | 1 |
| 90 | 1 | 1 |
| 105 | 2 | 2 |
| 120 | 15 | 13 |
| 135 | 28 | 24 |
| 150 | 44 | 37 |
| 165 | 56 | 47 |
| 180 | 66 | 55 |
| 195 | 68 | 57 |
| 210 | 71 | 60 |
| 225 | 82 | 69 |
| 240 | 85 | 71 |
| 255 | 91 | 76 |
| 270 | 92 | 77 |
| 285 | 94 | 79 |
| 300 | 97 | 82 |

The example shows that a solution with 1.8 g/l of added potassium chloride in sea water has a considerable effect on the ability of salmon lice to stay on the fish.

### Example 3

The same quality of sea water and the same method as in Example 1 were used. A total of 14 salmon were infected with 137 adult male lice and female lice. The salmon weighed between 125 and 510 g. The average weight was 230 g. The preparation for use as a medicament for the topical treatment of fish had an addition of 2.0 g KCl per litre of sea water. This corresponds to an addition of 1.05 g of potassium and 0.95 g of chlorine per litre of sea water. The salinity in the trial was thereby 36.5 ppt. The preparation solution of the trial contained approximately 1,424 mg/l K⁺ and 19,680 mg/l Cl⁻.

The trial lasted 5 hours.

The results are shown in Table 3.

**Table 3**

| Observation time (min) | Cumulative number fallen off (n) | Cumulative number fallen off (%) |
|---|---|---|
| 60 | 1 | 1 |
| 75 | 7 | 5 |
| 90 | 18 | 13 |
| 105 | 37 | 27 |
| 120 | 68 | 50 |
| 135 | 84 | 61 |
| 150 | 93 | 68 |
| 165 | 99 | 72 |
| 180 | 102 | 74 |
| 195 | 106 | 77 |
| 210 | 109 | 80 |
| 225 | 114 | 83 |
| 240 | 114 | 83 |
| 255 | 116 | 85 |
| 270 | 119 | 87 |
| 285 | 120 | 88 |
| 300 | 123 | 90 |

The example shows that a solution with 2.0 g/l of added potassium chloride in sea water has a considerable effect on the ability of salmon lice to stay on the fish, even without any mechanical influence.

Examples 2 and 3 show that the salmon louse does not fall off the fish until after about one hour of exposure. It is assumed that an elevated level of potassium in sea water has a paralyzing effect on the salmon louse, but not a lethal effect. The time of exposure until 50 % of the salmon lice had fallen off was 172 minutes, 171 minutes and 120 minutes, respectively, in the examples 1-3.

When known pharmaceuticals, including hydrogen peroxide, are used for bath treatment of salmonoids infected with salmon lice, a reduction of 90 % of the adult lice is held to be a good and satisfactory result of treatment.

## Claims

1. A preparation for use as a medicament for the topical treatment of fish infected with an external parasite, **characterized in that** the preparation consists of sea water to which a potassium compound has been added,
wherein the potassium compound is added in an amount corresponding to between 0.78 g of potassium ions per litre of said sea water and 1.21 g of potassium ions per litre of said sea water,
wherein the salinity of said sea water is between 20 ppt and 40 ppt, wherein said sea water is one of:
- natural sea water;
- natural sea water thinned with fresh water; and
- synthetic sea water containing at least chlorine, sodium magnesium, sulphur and potassium in approximately the same proportions as in natural sea water.

2. The preparation for use according to claim 1, wherein the potassium compound is a potassium salt.

3. The preparation for use according to claim 2, wherein the potassium salt is potassium chloride.

4. The preparation for use according to any one of the preceding claims, wherein the sea water comprises a sea water with a salinity of between 30 ppt and 38 ppt.

5. The preparation for use according to any one of claims 1 to 3, wherein extra salt which does not contain potassium, has been added to the sea water to a salinity of between 35 ppt and 40 ppt.

6. The preparation for use according to any one of the preceding claims, wherein the external parasite consists of a crustacean parasite.

7. The preparation for use according to claim 6, wherein the crustacean parasite consists of a salmon louse.

8. The preparation for use according to any one of claims 1-5, wherein the fish consists of a marine fish.

9. The preparation for use according to any one of claims 1-5, wherein the fish consists of a salmonoid.

## Patentansprüche

1. Präparat zur Verwendung als ein Medikament für die äussere Behandlung von Fischen, welche mit einem externen Parasiten infiziert sind, **dadurch gekennzeichnet, dass** das Präparat aus Meerwasser besteht, welchem eine Kaliumverbindung beigefügt wurde,
wobei die Kaliumverbindung in einer Menge entsprechend zwischen 0.78 g Kaliumionen pro Liter Meerwasser und 1.21 g Kaliumionen pro Liter Meerwasser beigefügt wird,
wobei der Salzgehalt des besagten Meerwassers zwischen 20 ppt und 40 ppt beträgt,
wobei das Meerwasser eines der Folgenden ist:
- natürliches Meerwasser;
- mit Frischwasser verdünntes natürliches Meerwasser, und
- synthetisches Meerwasser, mindestens aufweisend Chlor, Natrium, Magnesium, Schwefel und Kalium in ungefähr den gleichen Anteilen wie in natürlichem Meerwasser.

2. Präparat zur Verwendung gemäss Anspruch 1, wobei die Kaliumverbindung ein Kaliumsalz ist.

3. Präparat zur Verwendung gemäss Anspruch 2, wobei das Kaliumsalz Kaliumchlorid ist.

4. Präparat zur Verwendung gemäss einem der vorhergehenden Ansprüche, wobei das Meerwasser ein Meerwasser mit einem Salzgehalt von zwischen 30 ppt und 38 ppt enthält.

5. Präparat zur Verwendung gemäss einem der Ansprüche 1 bis 3, wobei zusätzliches Salz, welches kein Kalium enthält, zum Meerwasser hinzugefügt wurde, bis zu einem Salzgehalt von zwischen 35 ppt und 40 ppt.

6. Präparat zur Verwendung gemäss einem der vorhergehenden Ansprüche, wobei der externe Parasit aus einem Krustentier-Parasit besteht.

7. Präparat zur Verwendung gemäss Anspruch 6, wobei der Krustentier-Parasit eine Lachslaus ist.

8. Präparat zur Verwendung gemäss einem der Ansprüche 1 bis 5, wobei der Fisch ein Meeresfisch ist.

9. Präparat zur Verwendung gemäss einem der Ansprüche 1 bis 5, wobei der Fisch ein Lachsfisch ist.

## Revendications

1. Une préparation pour l'utilisation comme médicament pour le traitement topique de poissons infectés par un parasite externe, **caractérisée en ce que** la préparation consiste en de l'eau de mer à laquelle un composé de potassium a été ajouté,
dans laquelle le composé de potassium est ajouté en une quantité correspondant à entre 0,78 g d'ions de potassium par litre d'eau de mer et 1,21 g d'ions de potassium par litre d'eau de mer,
dans laquelle la salinité de ladite eau de mer se situe entre 20 ppt et 40 ppt,
dans laquelle ladite eau de mer est une parmi:
- l'eau de mer naturelle,
- l'eau de mer naturelle diluée avec de l'eau douce; et
- l'eau de mer synthétique contenant au moins du chlore, du sodium, du magnésium, du soufre et du potassium dans des proportions approximativement les mêmes que dans l'eau de mer naturelle.

2. La préparation pour l'utilisation selon la revendication 1, dans laquelle le composé de potassium est un sel de potassium.

3. La préparation pour l'utilisation selon la revendication 2, dans laquelle le sel de potassium est le chlorure de potassium.

4. La préparation pour l'utilisation selon l'une quelconque des revendications précédentes, dans laquelle l'eau de mer comprend une eau de mer avec une salinité se situant entre 30 ppt et 38 ppt.

5. La préparation pour l'utilisation selon l'une quelconque des revendications 1 à 3, dans laquelle du sel supplémentaire, qui ne contient pas de potassium, a été ajouté à l'eau de mer jusqu'à une salinité se situant entre 35 ppt et 40 ppt.

6. La préparation pour l'utilisation selon l'une quelconque des revendications précédentes, dans laquelle le parasite externe consiste en un parasite crustacé.

7. La préparation pour l'utilisation selon la revendication 6, dans laquelle le parasite crustacé consiste en un pou du saumon.

8. La préparation pour l'utilisation selon l'une quelconque des revendications 1 à 5, dans laquelle le poisson est un poisson marin.

9. La préparation pour l'utilisation selon l'une quelconque des revendications 1 à 5, dans laquelle le poisson est un salmonidé.
